# EUROPEAN PATENT SPECIFICATION

(11) **EP 1 420 692 B1**
(45) Date of publication and mention of the grant of the patent: **26.07.2006**
(21) Application number: 02763147.2
(22) Date of filing: 26.08.2002
(51) Int. Cl.: A61B 5/08, A61M 16/00, G01N 21/85

(54) **DEVICE FOR QUANTITATIVE ANALYSIS OF RESPIRATORY GASES, COMPRISING A PASSIVE RESPIRATORY GAS HUMIDIFYER, WHERE RAYS OF LIGHT ARE TRANSMITTED THROUGH A DEHUMIFIED GAS FLOW**
VORRICHTUNG FÜR DIE QUANTITATIVE ANALYSE VON ATEMGASEN, UMFASSEND EINEN PASSIVEN ATEMGASBEFEUCHTER, IN DER LICHTSTRAHLEN DURCH EINEN ENTFEUCHTETEN GASFLUSS GELEITET WERDEN
DISPOSITIF D'ANALYSE QUANTITATIVE DE GAZ RESPIRATOIRES COMPRENANT UN HUMIDIFICATEUR DE GAZ RESPIRATOIRE PASSIF, ET DANS LEQUEL DES FAISCEAUX LUMINEUX SONT EMIS A TRAVERS UN FLUX DE GAZ DESHUMIDIFIE

(30) Priority: 28.08.2001 SE 0102860
(43) Date of publication of application: 26.05.2004
(73) Proprietor: Phase-In AB, 111 73 Stockholm (SE)
(72) Inventor: ECKERBOM, Anders, S-185 41 VAXHOLM (SE)
(74) Representative: Karlström, Lennart
(86) International application number: PCT/SE2002/001526
(87) International publication number: WO 2003/017836

(56) References cited:
- EP-A1- 0 536 978
- US-A- 4 648 396
- US-A- 5 109 471
- US-A- 6 095 986

## Description

The present invention relates to an arrangement pertaining to the quantitative analysis of respiratory gases flowing to and from a patient connected to a respirator for breathing assistance.

With regard to gas analysis carried out in connection with respiratory care, a distinction is made between two principle types of gas analysers, i.e. between lateral flow measuring analysers and main flow measuring analysers. The lateral flow measuring analysers take a minor sample flow from the respiratory circuit of a patient to an adjacent instrument in which the actual gas analysis takes place, whereas the main flow measuring analysers calculate the gas concentrations directly in the respiratory circuit of the patient. The main flow measuring analyser is normally placed as close as possible to the patient's mouth or trachea, for reasons of accuracy.

The main flow measuring analysers can be made less expensive, smaller, more energy-lean and more responsive than the lateral flow measuring analysers, since the need for sample flow handling (pumps, hoses, etc.) is obviated. Consequently, the main flow measuring gas analysers are preferred over the lateral flow measuring analysers. However, the use of main flow measuring analysers has been restricted essentially to emergency care due to technical problems, primarily caused by the presence of moisture and bacteria in the patient's respiratory circuit.

Main flow measuring gas analysis can be effected in accordance with different measuring or assaying principles. Gas analysis, however, is most usually effected by non-dispersive spectroscopy. This measuring principle is based on the fact that many gases absorb infrared energy at a wavelength specific for the substance concerned. Main flow measuring gas analysers based on gas analysis by non-dispersive spectroscopy thus measure the absorption of light at specific wavelengths directly in the patient's respiratory circuit. To this end, it is necessary for the infrared radiation to pass through an entrance window and an exit window, where said windows must be adapted to allow light of the desired wavelength to pass freely through the patient's respiratory circuit.

To avoid the airways of long-term patients from drying out, the respiratory gases of such patients are often heated and actively moisturised. Short-term patients, however, often manage without active moisturisation, although such patients also exhale body-heated, moist gas. Water vapour will then readily condense from a gas sample onto the window of the main flow measuring gas analyser. Such condensation can lead to signal losses and therewith to subsequent errors in the determined composition of the gas sample.

Moisture problems in respect of main flow measuring gas analysers are solved traditionally by heating said windows to a temperature that prevents condensation of moisture onto the windows. Although such solutions can be effective, they also have several drawbacks. Perhaps the most decisive drawback is that heating consumes energy, which makes it difficult to integrate such analysers in transportable battery-powered instruments. Moreover, it takes time for the heat to build up after the instrument has been switched on, meaning that the instrument must be activated some time before it is taken into use. The heat can also constitute a risk of burning the patient.

A more refined solution to the moisture problem is based on treating the windows chemically, so as to avoid the risk of condensation. Such chemicals are marketed, inter alia, by ICI Speciality Chemicals under the trade name "Atmer 385". Although this solution avoids all the drawbacks associated with heating of the windows, it is, unfortunately, often impossible to fully eliminate the moisture problems with this method. Patients whose breath needs to be moistened actively are therewith often difficult to supervise.

The document US-A-6 095 986 discloses a gas analyser that includes an adapter having a connection for a respirator, and also a connection for a hose that leads to the patient, wherein a measuring head connection is provided between the passive respiratory humidifier and the respirator connection, and wherein the measuring head connection has two windows through which light beams from the measuring head can pass.

Accordingly, the object of the present invention is to provide a novel arrangement with which the aforesaid problems can be avoided.

This object is achieved in accordance with the invention with an arrangement as defined in claim 1.

According to one particular embodiment of the inventive gas analyser, the analyser is designed to enable it to also be used for measuring oxygen-gas concentrations. Further preferred embodiments are defined in the dependent claims.

The invention will now be described in more detail with reference to a non-limiting embodiment thereof and also with reference to the accompanying drawings, in which **Fig. 1** is a perspective, schematic view of inventive arrangement with an associated measuring head; **Fig. 2** is a schematic illustration of a patient connection to a respirator while using the inventive arrangement; and **Fig. 3** is a schematic sectional view of an adapter according to the invention.

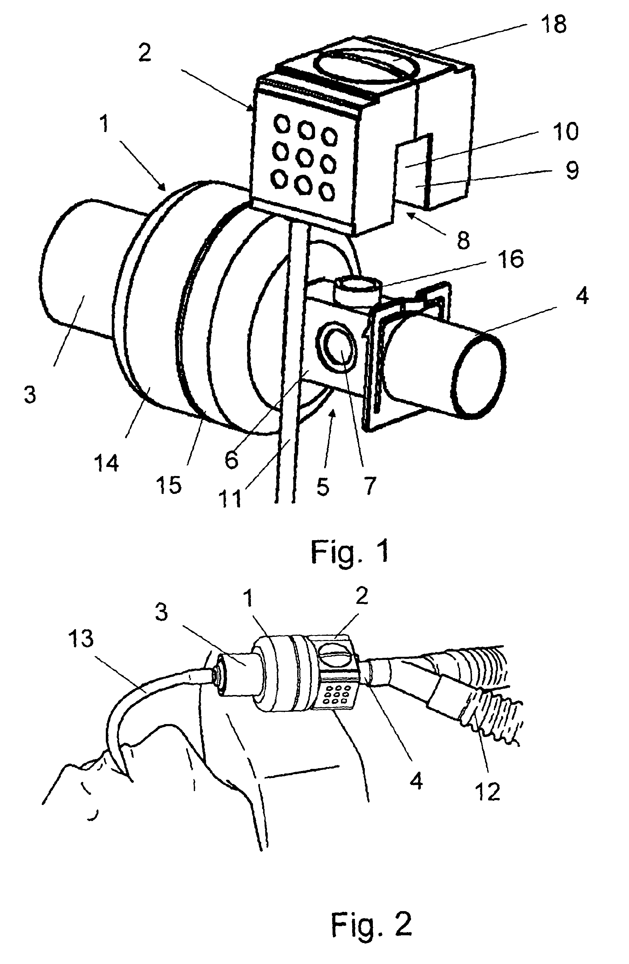

Thus, Fig. 1 shows a gas analyser constructed in accordance with the invention and comprising an adapter 1 and an associated measuring head 2. The adapter 1 has essentially the form of an elongate tube made, for instance, of a plastic material. The adapter 1 has at one end a connector 3 for a hose that leads to the patient. The other end of the adapter carries a connector 4 for a respirator or the like. Located between the two connectors 3, 4 on the adapter 1 is a central portion 5 which is designed to accommodate the measuring head. To this end, the central portion 5 includes two mutually opposing planar surfaces 6, each of which includes a respective window 7 comprised of transparent film.

The measuring head 2 includes a central aperture 8 which extends from one side of the measuring head so as to enable the measuring head to be pushed over the central portion 5 of the adapter. To this end, the aperture is provided with two mutually opposing, generally planar and mutual parallel surfaces 9 that face inwardly towards the aperture. Respective planar surfaces 9 on the measuring head 2 are provided with a light transmitter and a light receiver 10 for transmitting and receiving infrared light respectively. The light transmitter and light receiver are connected by a signal cable 11 to a measuring instrument that analyses the signals obtained from the receiver. The planar surfaces 9 on the measuring head 2 and the planar sides 6 of the central portion 5 of the adapter 1 are mutually designed and dimensioned so that the measuring instrument 2 will be positioned precisely when mounted on the adapter 1, so that light emitted by the light transmitter 10 is able to pass through the central portion 5 of the adapter and through its window 7, and reach the light receiver without being influenced by anything other than that which passes through the interior of the central portion 5 of the adapter.

As will be seen from Fig. 1, the adapter 1 also includes a passive respiratory humidifier or breath moistener 14 between its central portion 5 containing the planar sides 6 for receiving the measuring head and the windows 7 on the planar surfaces, and the connection 3 for connecting the adapter to the patient hose. This passive humidifier may be a so-called HCH, Hygroscopic Condensation Humidifier, or an HME, Heat Moisture Exchanger, of the types generally used in respiratory care. These devices moisturise the respiratory gases by capturing moisture, and to some extent also heat, as the patient breathes out, and then return the moisture to the inspiration air as the patient breathes in. Because the passive respiratory humidifier 14 is situated between the patient hose connection 3 and the central portion 5 of the adapter, the expiration gases will be dehumidified when entering the central portion, where the windows 7 are situated, therewith preventing the occurrence of condensation on said windows and also enabling the expiration gas flowing through said central portion 5 to be analysed in a known manner with the aid of the measuring head 2. The passive humidifier 14 is placed in the adapter in the form of a piece of wadding or a roll impregnated with a hygroscopic salt and inserted through the open end of the connector 3.

In addition to the humidifier 14, the adapter 1 may also include bacteria filter 15 situated between the humidifier 14 and the central portion 5. The filter 15 enables bacteria to be removed from the expiration gas, so that, e.g., the oxygen gas concentration can be measured with the aid of a fuel cell without danger of cross contamination between different patients.

As indicated above, a fuel cell can be used in the central portion 5 of the adapter for measuring the oxygen gas concentration of the expiration gas. To this end, a connection 16 to which a fuel cell can be connected may be provided in a side wall of the central portion 5 that lacks a window 7.

Fig. 2 illustrates a patient connected to a respirator with the aid of an arrangement according to the invention. The figure shows that respiratory hoses 12 are connected to the adapter connection 4, and that a patient hose 13 is connected to the patient from the adapter connection 3.

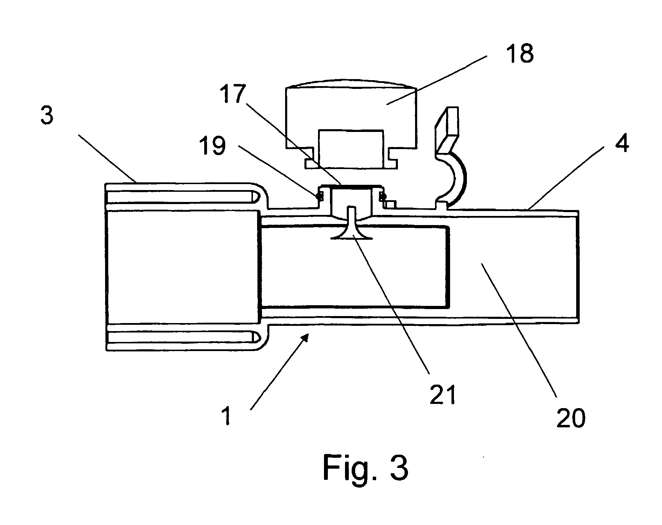

Fig. 3 shows how a fuel cell 18 provided with O-rings 19 can be fastened to the central portion 5 of an adapter. Also shown in the figure is the internal channel 20 in the central portion 5 through which the respiratory gases flow to and from the patient. The internal channel may be provided conveniently with a flow directing means 21 for guiding part of the respiratory gases towards the fuel cell 18 and thereby reducing the step response of the oxygen gas measuring process.

The connection 16 may be provided with a bacterial filter 17, so as to provide further protection against cross-contamination.

The inventive adapter may conveniently be injection-moulded from plastic material and therewith be produced for one-time use at a relatively low cost. The measuring head casing may also be produced from a plastic material although not for one-time use, since the measuring head is used together with the measuring instrument and is not affected or contaminated by the respiratory gases.

## Claims

1. An arrangement for the quantitative analysis of respiratory gases flowing to and from a patient connected to a respirator for breathing assistance, said arrangement comprising an adapter (1) that has a connector (4) for connection to a respirator, and a connector (3) for connection to a hose (13) leading to the patient, wherein the adapter includes a passive respiratory gas humidifier (14) situated between the respirator connector (4) and the connector (3) for connecting said hoses to the patient; and a connection for a gas analyser measuring head (2) is provided between the passive humidifier (14) and the respirator connector (4), the measuring head connector including two windows (7) through which light rays from the measuring head (2) can pass.

2. An arrangement according to Claim 1, **characterised in that** the measuring head (2) connection includes two mutually opposing planar sides (6) in which said windows (7) are located; and **in that** the measuring head (2) includes a central aperture (8) that has two mutually facing planar surfaces (9) for sealing attachment of the measuring head over the planar sides (6) of the measuring head connector.

3. An arrangement according to Claim 1 or 2, **characterised in that** the passive humidifier (14) is placed in the adapter in the form of wadding or a roll impregnated with a hygroscopic salt.

4. An arrangement according to any one of the preceding Claims, **characterised in that** the arrangement includes a bacterial filter (15) disposed between the respiratory gas humidifier (14) and said central portion (5).

5. An arrangement according to any one of the preceding Claims, **characterised in that** the arrangement includes in the central portion (5) of the adapter a connection (16) for a fuel cell (18) for measuring the oxygen gas content of the respiratory gases.

6. An arrangement according to Claim 5, **characterised in that** the fuel cell connection (16) includes a bacterial filter (17).

7. An arrangement according to Claim 5 or 6, **characterised in that** the adapter (1) includes flow directing means (21) for guiding part of the respiratory gases towards the fuel cell (18).

## Patentansprüche

1. Vorrichtung für die quantitative Analyse von Atemgasen, welche zu und von einem Patienten fließen, der mit einem Beatmungsgerät zur Unterstützung der Beatmung verbunden ist, wobei die Vorrichtung einen Adapter (1) aufweist, der einen Verbinder (4) zur Verbindung mit einem Beatmungsgerät und einen Verbinder (3) zur Verbindung mit einem Schlauch (13), welcher zu dem Patienten führt aufweist, wobei der Adapter einen passiven Beatmungsgasbefeuchter (14) aufweist, der zwischen dem Verbinder (4) des Beatmungsgeräts und dem Verbinder (3) zur Verbindung der Schläuche mit dem Patienten angeordnet ist, und mit einer Verbindung für einen Gasanalysemesskopf (2), welcher zwischen dem passiven Befeuchter (14) und dem Verbinder (4) für das Beatmungsgerät angeordnet ist, wobei der Verbinder für den Messkopf zwei Fenster (7) aufweist, durch welche Lichtstrahlen von dem Messkopf (2) hindurchgehen können.

2. Anordnung nach Anspruch 1, **dadurch gekennzeichnet, dass** die Verbindung des Messkopfes (2) zwei einander gegenüberliegende ebene Seiten (6) aufweist, in welchen die genannten Fenster (7) angeordnet sind, und dass der Messkopf (2) eine Zentralöffnung (8) aufweist, welche zwei zueinander zeigende ebene Oberflächen (9) zur dichtenden Befestigung des Messkopfes über den ebenen Seiten (6) des Verbinders des Messkopfes aufweist,

3. Anordnung nach Anspruch 1 oder 2, **dadurch gekennzeichnet, dass** der passive Befeuchter (14) in dem Adapter in Form einer Wattierung oder einer Rolle, welche mit einem hygroskopischen Salz imprägniert ist, angeordnet ist,

4. Anordnung nach einem der vorhergehenden Ansprüche, **dadurch gekennzeichnet, dass** die Anordnung einen Bakterienfilter (15) aufweist, welcher zwischen dem Beatmungsgasbefeuchter (14) und dem Zentralabschnitt (5) angeordnet ist.

5. Anordnung nach einem der vorhergehenden Ansprüche, **dadurch gekennzeichnet, dass** die Anordnung im Zentralabschnitt (5) des Adapters eine Verbindung (16) für eine Betriebsstoffzelle (18) zum Messen des Sauerstoffgasgehalts des Beatmungsgases aufweist.

6. Anordnung nach Anspruch 5, **dadurch gekennzeichnet, dass** die Verbindung (16) für die Betriebsstoffzelle einen Bakterienfilter (17) aufweist,

7. Anordnung nach Anspruch 5 oder 6, **dadurch gekennzeichnet, dass** der Adapter (1) eine Strömungsausrichtungseinrichtung (21) zum Leiten eines Teils der Beatmungsgase zu der Betriebsstoffzelle (18) aufweist.

## Revendications

1. Agencement d'analyse quantitative de gaz respiratoire circulant vers et à partir d'un patient relié à un respirateur pour assister la respiration, ledit agencement comprenant un adaptateur (1) qui présente un connecteur (4) pour la connexion avec un respirateur et un connecteur (3) pour la connexion avec un tuyau (13) menant vers le patient, dans lequel l'adaptateur comprend un humidificateur de gaz respiratoire passif (14) situé entre le connecteur de respirateur (4) et le connecteur (3) pour connecter lesdits tuyaux avec le patient, une connexion pour une tête de mesure d'analyseur de gaz (2) étant prévue entre l'humidificateur passif (14) et le connecteur de respirateur (4), le connecteur de tête de mesure présentant deux fenêtres (7) à travers lesquelles des rayons lumineux émanant de la tête de mesure (2) peuvent passer.

2. Agencement selon la revendication 1, **caractérisé en ce que** la connexion de tête de mesure (2) comprend deux côtés planes mutuellement opposés (6) dans lesquels les deux fenêtres (7) sont situées et **en ce que** la tête de mesure (2) comprend une ouverture centrale (8) qui présente deux surfaces planes (9) mutuellement en regard pour une fixation étanche de la tête de mesure au-dessus des côtés planes (6) du connecteur de tête de mesure.

3. Agencement selon la revendication 1 ou 2, **caractérisé en ce que** l'humidificateur passif (14) est placé dans l'adaptateur sous forme de rembourrage ou de rouleau imprégnés d'un sel hygroscopique.

4. Agencement selon l'une quelconque des revendications précédentes, **caractérisé en ce que** l'agencement comprend un filtre antibactérien (15) disposé entre l'humidificateur de gaz respiratoire (14) et ladite portion centrale (5).

5. Agencement selon l'une quelconque des revendications précédentes, **caractérisé en ce que** l'agencement comprend dans la portion centrale (6) de l'adaptateur une connexion (16) pour une cellule à combustible (18) pour mesurer le contenu de gaz d'oxygène des gaz respiratoires.

6. Agencement selon la revendication 5, **caractérisé en ce que** la connexion de cellule à combustible (16) comprend un filtre antibactérien (17).

7. Agencement selon les revendications 5 ou 6, **caractérisé en ce que** l'adaptateur (1) comprend un moyen d'orientation de flux (21) pour guider une partie des gaz respiratoires vers la cellule à combustible (18).
